# DEMANDE DE BREVET EUROPEEN

(11) **EP 1 881 029 A2**
(43) Date de publication de la demande: **23.01.2008**
(21) Numéro de dépôt: 07117694.5
(22) Date de dépôt: 07.10.2004
(51) Int. Cl.: C08L 33/00

(54) **Nouveau latex inverse concentré, procédé pour sa préparation et utilisation dans l'industrie**

(30) Priorité: 22.10.2003 FR 0350717
(62) Demande divisionnaire de: 04805735.0
(71) Demandeur: SOCIETE D'EXPLOITATION DE PRODUITS POUR LES INDUSTRIES CHIMIQUES, S.E.P.P.I.C., 75007 Paris (FR)
(72) Inventeur: BRAUN, Olivier, 81100, CASTRES (FR); MALLO, Paul, 78290, CROISSY-SUR-SEINE (FR)
(74) Mandataire: Conan, Philippe Claude

(57) **Abrégé**

Composition sous forme d'un latex inverse comprenant :
a) de 50 % en poids à 80 % d'au moins un polymère organique (P) choisis parmi les copolymères réticulés de l'acide 2-méthyl 2-[(1-oxo 2-propènyl) amino] 1-propanesulfonique partiellement salifié sous forme de sel de sodium et de l'acrylate de 2-hydroxy éthyle ;
b) de 5 % en poids à 10 % d'un système émulsionnant (S₁) de type eau dans huile (E/H),
c) de 5 % en poids à 45 % en poids d'au moins une huile, et
d) de 0 % à 5% d'eau.
Procédé pour sa préparation et utilisation.

## Description

La présente demande concerne des latex inverse eau dans huile épaississants, leur procédé de préparation et leur application en tant qu'épaississant et/ou émulsionnant dans les produits industriels, les produits de soins de la peau et des cheveux ou pour la fabrication de préparations cosmétiques, dermopharmaceutiques ou pharmaceutiques.

Les latex inverses de polymères de l'acide l'acide 2-méthyl 2-[(1-oxo 2-propènyl) amino] 1-propanesulfonique (dénommé aussi acide 2-acrylamido 2-méthyl propanesulfonique, ATBS ou AMPS) partiellement ou totalement salifié ainsi que leur utilisation en cosmétique et/ou pharmacie ont fait de nombreuses demandes de brevet. Cependant la présence de quantités importantes d'eau et d'huile représente un inconvénient non négligeable en termes de volume, de coût et parfois de risques accrus et/ou d'effets toxiques.

Des solutions ont donc été développées pour augmenter la concentration en polymères dans les latex finaux par exemple en soumettant le milieu réactionnel en fin de polymérisation, à une étape de distillation sous vide pour enlever une partie plus ou moins importante d'eau et d'huile. Cette distillation est cependant délicate à mettre en oeuvre car elle induit souvent une déstabilisation du latex inverse qu'il faut contrer par l'addition préalable d'agents stabilisants. Les demandes de brevet européen EP 0 161 038 et EP 0 126 528 ainsi que la demande de brevet britannique GB 1 482 515 divulguent une telle utilisation de polymères stabilisants.

Mais ceux-ci contiennent des alcools ou des glycols qui induisent des problèmes environnementaux. De plus il se produit parfois une prise en masse du milieu réactionnel lors de l'étape de distillation sans que ce phénomène n'ait jamais vraiment été expliqué, mais dont la conséquence certaine est la destruction du lot de latex inverse et un nettoyage pénible et coûteux du réacteur. Enfin, même quand la distillation se déroule correctement, les latex inverses obtenus s'inversent souvent difficilement, ils ont une viscosité élevée et présentent parfois en leur sein des micro-gels. Ces inconvénients interdisent donc leur utilisation dans la fabrication de formulations cosmétiques et ou d'impression textile.

C'est pourquoi la demanderesse s'est attachée à mettre au point des latex inverses concentrés, c'est à dire comprenant au moins 50 % en poids de polymère et moins de 5 % en poids d'eau dépourvus de tels inconvénients.

Selon un premier aspect, l'invention a pour objet une composition sous forme d'un latex inverse comprenant :
a) de 50 % en poids à 80 % d'au moins un polymère organique (P) choisi parmi les copolymères réticulés de l'acide 2-méthyl 2-[(1-oxo 2-propènyl) amino] 1-propanesulfonique partiellement salifié sous forme de sel de sodium et de l'acrylate de 2-hydroxy éthyle,
b) de 5 % en poids à 10 % d'un système émulsionnant (Si) de type eau dans huile (E/H),
c) de 5 % en poids à 45 % en poids d'au moins une huile, et
d) de 0 % à 5 % d'eau.

La composition telle que définie précédemment contient, soit un seul polymère (P), soit un mélange de polymères (P) différents.

Dans la composition telle que définie ci-dessus, le système émulsionnant (Si) de type eau dans huile (E/H) est constitué soit d'un seul tensioactif soit d'un mélange de tensioactifs à condition que ledit mélange ait une valeur de HLB suffisamment faible pour induire des émulsions eau dans huile. Comme agent émulsionnant de type eau - dans huile, il y a par exemple les esters de sorbitan, comme le l'oléate de sorbitan, comme celui commercialisé par la société SEPPIC sous le nom MONTANE™ 80, l'isostéarate de sorbitan, comme celui commercialisé par la société SEPPIC sous le nom MONTANE™ 70 ou le sesquioléate de sorbitan comme celui commercialisé par la société SEPPIC sous le nom MONTANE™ 83. Il y aussi certains esters de sorbitan polyéthoxylés, par exemple le monooléate de sorbitan pentaéthoxylé comme celui commercialisé par la société SEPPIC sous le nom MONTA-NOX™ 81 ou l'isostéarate de sorbitan pentaéthoxylé comme celui commercialisé sous le nom MONTANOX™ 71 par la société SEPPIC. Il y a encore l'alcool oléocétylique diéthoxylé comme celui commercialisé sous le nom SIMULSOL™ OC 72 par la société SEPPIC, l'acrylate de lauryle tétraéthoxylé comme celui commercialisé sous le nom BLEM-MER™ ALE 200 ou les polyesters de poids moléculaire compris entre 1000 et 3000, produits de la condensation entre un acide poly(iosbutènyl) succinique ou son anhydride et un polyéthylène glycol, tels que l'HYPERMER™ 2296 commercialisé par la société UNICHEMA ou enfin les copolymères blocks de poids moléculaire compris entre 2500 et 3500, comme l'HYPERMER™ B246 commercialisé par la société UNICHEMA ou le SIMA-LINE™ IE 200 commercialisé par la société SEPPIC.

Par polymère réticulé, on désigne pour (P), un polymère non linéaire se présentant à l'état de réseau tridimensionnel insoluble dans l'eau, mais gonflable à l'eau et conduisant donc à l'obtention d'un gel chimique. Il est plus particulièrement réticulé avec un composé diéthylènique ou polyéthylènique dans la proportion molaire exprimée par rapport aux monomères mis en oeuvre, de 0,005% à 1%, et de préférence de 0,01 % à 0,2 % et, plus particulièrement de 0,01 % à 0,1 %. De préférence l'agent de réticulation et/ou l'agent de ramification est choisi parmi le diméthacrylate d'éthylèneglycol, le diacrylate de diéthylèneglycol, le diallyloxyacétate de sodium, le diacrylate d'éthylèneglycol, le diallyl urée, le triallylamine, le triméthylol propanetriacrylate ou le méthylène-bis-(acrylamide).

Selon un premier mode particulier de la présente invention, la composition telle que définie précédemment, comprend au moins de 60 % en poids et au plus 70 % en poids de polymère (P).

Selon un deuxième mode particulier de la présente invention, la composition telle que définie précédemment, comprend en outre jusqu'à 5 % de son poids d'un système émulsionnant (S₂) de type huile dans eau (H/E).

Par " agent émulsifiant du type huile dans eau ", on désigne des agents émulsifiants possédant une valeur de HLB suffisamment élevée pour fournir des émulsions huile dans l'eau tels que les esters de sorbitan éthoxylés comme l'oléate de sorbitan polyéthoxylé avec 20 moles d'oxyde d'éthylène, commercialisé par la société SEPPIC sous le nom de MON-TANOX™ 80, le laurate de sorbitan polyéthoxylé avec 20 moles d'oxyde d'éthylène, commercialisé par la société SEPPIC sous le nom de MONTANOX™ 20, l'huile de ricin polyéthoxylé avec 40 moles d'oxyde d'éthylène commercialisé sous le nom SIMULSOL™ OL50, l'alcool oléodécylique décaéthoxylé, commercialisé par la société SEPPIC sous le nom SIMULSOL™ OC 710, l'alcool laurique heptaéthoxylé commercialisé sous le nom SIMULSOL™ P7, le nonylphénol décaéthoxylé commercialisé sous le nom de SYNPE-RONIC™ NP-10 ou les hexaoléates de sorbitan polyéthoxylés commercialisés par la société ATLAS sous les nom G-1086 et G-1096.

Dans la composition objet de la présente invention, la phase huile est constituée soit par une huile minérale commerciale contenant des hydrocarbures saturés comme les paraffines, les isoparaffines, les cycloparaffines, présentant à température ambiante, une densité entre 0,7 et 0,9 et un point d'ébullition supérieur à environ 250°C, telle que par exemple le MARCOL™ 52 commercialisés par EXXON CHEMICAL, soit par une huile végétale comme le squalane d'origine végétale, soit une huile de synthèse tel que le polyisobutène hydrogéné ou le polydécène hydrogéné, soit par un mélange de plusieurs de ces huiles. Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993). La composition selon l'invention peut également contenir divers additifs tels que des agents complexants, des agents de transfert ou des agents limiteurs de chaîne.

Selon un autre aspect de la présente invention, celle-ci a pour objet un procédé de préparation de la composition telle que définie précédemment, caractérisé en ce que :
a) l'on émulsionne une phase aqueuse (A) contenant les monomères et les éventuels additifs hydrophiles, dans une phase organique (O) contenant, le système tensioactif(S₁), un mélange constitué de l'huile destinée à être présente dans la composition finale et d'une huile volatile et les éventuels additifs hydrophobes,
b) l'on amorce la réaction de polymérisation par introduction dans l'émulsion formée en a), d'un initiateur de radicaux libres puis on la laisse se dérouler, et
c) l'on concentre par distillation, le milieu réactionnel issu de l'étape b) jusqu'à élimination complète de ladite huile volatile.

Les huiles volatiles appropriées à la mise en oeuvre du procédé tel que défini ci-dessus, sont par exemple des isoparaffines légères comportant de 8 à 11 atomes de carbone comme par exemple ceux vendues sous les noms ISOPAR™ G, ISOPAR™ L ou ISO-PAR™ H ou ISOPAR™ J.

Selon une mise en oeuvre préférée du procédé tel que défini précédemment, la réaction de polymérisation est amorcée par un couple oxydoréducteur, tel que le couple hydroperoxyde de cumène - métabisulfite de sodium, à une température inférieure ou égale à 10°C, puis conduite soit de manière quasi-adiabatique jusqu'à une température supérieure ou égale à 40°C, plus particulièrement supérieure ou égale à 50°C, soit en contrôlant l'évolution de la température.

Lorsque l'étape c) est terminée, on introduit si désiré un ou plusieurs agents émulsifiants de type huile dans eau à une température inférieure à 50°C.

L'invention a aussi pour objet l'utilisation de la composition telle que définie précédemment pour préparer une composition topique cosmétique, dermopharmaceutique ou pharmaceutique.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile dans eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau et des muqueuses. Dans ce dernier cas, la composition topique comporte alors un principe actif qui peut par exemple consister en un agent anti-inflammatoire, un myorelaxant, un antifongique ou un antibactérien.

Lorsque la composition topique est utilisée en tant que composition cosmétique destinée à être appliquée sur la peau ou les muqueuses, elle peut ou non comporter un principe actif, par exemple un agent hydratant, un agent bronzant, un filtre solaire, un antirides, un agent à visée amincissante, un agent antiradicalaire, un agent antiacnéique ou un antifongique.

Une composition topique selon l'invention comporte habituellement entre 0,1 % et 10 % en poids de l'agent épaississant défini ci-dessus. Le pH de la composition topique est de préférence supérieur ou égal à 5.

La composition topique peut en outre comporter des composés classiquement compris dans ce type de compositions, par exemple des parfums, des conservateurs, des colorants, des émollients ou des tensioactifs.

Selon encore un autre aspect, l'invention concerne l'utilisation du nouvel agent épaississant conforme à l'invention, mentionné ci-dessus, pour épaissir et émulsionner une composition topique comprenant au moins une phase aqueuse.

La composition selon l'invention est un substitut intéressant à celles vendues sous les noms SEPIGEL™ 305, SEPIGEL™ 501, SIMULGEL™ EG, SIMULGEL™ NS ou SIMULGEL™ 600 par la demanderesse, car elle présente aussi une bonne compatibilité avec les autres excipients utilisés pour la préparation de formulations telles que les laits, les lotions, les crèmes, les savons, les bains, les baumes, les shampooings ou les après-shampooings. Elle peut aussi être mise en oeuvre avec lesdits SEPIGEL ou SIMULGEL.

Elle est notamment compatible avec les concentrés décrits et revendiqués dans les publications internationales WO 92/06778, WO 95/04592, WO 95/13863, WO 96/37285, WO 98/22207, WO 98/47610 ou dans FR 2734 496, avec les agents tensioactifs décrits dans WO 93/08204.

Elle est particulièrement compatible avec le MONTANOV™ 68, le MONTANOV™ 82, le MONTANOV™ 202 ou le SEPIPERL™ N. Elle peut également être utilisée dans des émulsions du type de celles décrites et revendiquées dans EP 0 629 396 et dans les dispersions aqueuses cosmétiquement ou physiologiquement acceptable avec un composé organopolysiloxane choisi, par exemple parmi ceux décrits dans WO 93/05762 ou dans WO 93/21316.

Elle peut également être utilisée pour former des gels aqueux à pH acide cosmétiquement ou physiologiquement acceptable, tels que ceux décrit dans WO 93/07856 ; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019. ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 195 23596 ou en association avec d'autres polymères épaississants.

La composition selon l'invention est également compatible avec les principes actifs tels que par exemple, les agents auto-bronzants comme le dihydroxyacétone (DHA) ou les agents anti-acné ; elle peut donc être introduite dans des compositions auto-bronzantes comme celles revendiquées dans EP 0 715 845, EP 0 604249, EP 0576188 ou dans WO 93/07902.

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou dans WO 98/09611.

Lorsque la composition telle que définie précédemment est destinée au traitement de cheveux, elle comprend plus particulièrement un latex inverse de polymère cationique objet de la présente invention.

Lorsque la composition telle que définie précédemment est destinée au traitement de la peau et/ou des muqueuses, elle comprend plus particulièrement un latex inverse de polymère anionique objet de la présente invention.

Les latex inverse objet de la présente invention peuvent être utilisés comme épaississant de pâtes d'impression textile

## Revendications

1. Composition sous forme d'un latex inverse comprenant :
a) de 50 % en poids à 80 % d'au moins un polymère organique (P), choisi parmi les copolymères réticulés de l'acide 2-méthyl 2-[(1-oxo 2-propènyl) amino] 1-propanesulfonique partiellement salifié sous forme de sel de sodium et de l'acrylate de 2-hydroxy éthyle ;
b) de 5 % en poids à 10 % d'un système émulsionnant (Si) de type eau dans huile (E/H),
c) de 5 % en poids à 45 % en poids d'au moins une huile, et
d) de 0 % à 5 % d'eau.

2. Composition telle que définie à la revendication 1, dans laquelle le polymère (P) est réticulé avec un composé diéthylènique ou polyéthylènique dans la proportion molaire exprimée par rapport aux monomères mis en oeuvre, de 0,005% à 1%, et de préférence de 0,01 % à 0,2 % et, plus particulièrement de 0,01 % à 0,1 %.

3. Composition telle que définie à la revendication 2, pour laquelle l'agent de réticulation et/ou l'agent de ramification est choisi parmi le diméthacrylate d'éthylèneglycol, le diacrylate de diéthylèneglycol, le diallyloxyacétate de sodium, le diacrylate d'éthylèneglycol, le diallyl urée, le triallylamine, le triméthylol propanetriacrylate ou le méthylène-bis-(acrylamide).

4. Composition telle que définie à l'une des revendications 1 à 3, comprenant de 60 % en poids à 70 % en poids de polymère (P).

5. Composition telle que définie à l'une de s revendications 1 à 4, comprenant en outre jusqu'à 5 % de son poids d'un système émulsionnant (S₂) de type huile dans eau (H/E).

6. Procédé de préparation de la composition telle que définie à l'une des revendications 1 à 5, **caractérisé en ce que** :
a) l'on émulsionne une phase aqueuse (A) contenant les monomères et les éventuels additifs hydrophiles, dans une phase organique (O) contenant, le système tensioactif (S₁), un mélange constitué de l'huile destinée à être présente dans la composition finale et d'une huile volatile et les éventuels additifs hydrophobes,
b) l'amorce la réaction de polymérisation par introduction dans l'émulsion formée en a), d'un initiateur de radicaux libres puis on la laisse se dérouler, et
c) l'on concentre par distillation, le milieu réactionnel issu de l'étape b), jusqu'à élimination complète de ladite huile volatile.

7. Procédé tel que défini à la revendication 6, dans lequel à l'issu de l'étape c), on introduit un ou plusieurs agents émulsifiants de type huile dans eau à une température inférieure à 50°C.

8. Utilisation de la composition telle que définie à l'une des revendications 1 à 5, comme épaississant et/ou émulsionnant de composition topique, cosmétique, dermopharmaceutique ou pharmaceutique.

9. Utilisation de la composition telle que définie à l'une des revendications 1 à 5, comme épaississant de pâtes d'impression textile.
